Europäisches Patentamt

European Patent Office

Office européen des brevets

(11) Numéro de publication: **0 421 867 A1**

# (12) DEMANDE DE BREVET EUROPEEN

(21) Numéro de dépôt: 90402720.8

(22) Date de dépôt: 02.10.90

(51) Int. Cl.5: **C11C 1/04, C12P 7/64**

(30) Priorité: 04.10.89 FR 8912980

(43) Date de publication de la demande:
10.04.91 Bulletin 91/15

(84) Etats contractants désignés:
AT BE CH DE DK ES FR GB GR IT LI LU NL SE

(71) Demandeur: MEDGENIX GROUP, S.A.
1, place d'Italie
B-4020 Liège(BE)

(72) Inventeur: Mendy, François
24 Quai du 4 Septembre
F-92100 Boulogne(FR)
Inventeur: Thonart, Philippe
20, rue de la Houlette
B-5850 La Bruyère(BE)
Inventeur: Debaty, Luc
102, rue des Golettes
B-5201 Tihange (Huy)(BE)
Inventeur: Delacroix, Dominique
11, avenue des Ramiers
B-1950 Kraainem(BE)

(74) Mandataire: Ahner, Francis et al
CABINET REGIMBEAU, 26, avenue Kléber
F-75116 Paris(FR)

(54) Procédé de production d'un mélange de glycérides enrichis en acides gamma linolénique et stéaridonique.

(57) La présente invention a pour objet un procédé de production par sélection d'un mélange de glycérides enrichis en acides gras essentiels gamma-linolénique et/ou stéaridonique sous forme estérifiée en position 1, 2 ou 3, de préférence 2, des glycérides ou d'une matière grasse telle qu'une huile végétale en contenant, caractérisé en ce que :

a) à partir d'un mélange de glycérides dont certains contiennent naturellement des acides gras gamma-linolénique et/ou stéaridonique sous forme estérifiée en position 1, 2 ou 3, de préférence 2, sur le glycéride, ou de l'huile en contenant, on hydrolyse ledit mélange ou l'huile en contenant avec une lipase dont la spécificité est de ne pas ou difficilement hydrolyser le lien ester des acides gras gamma-linolénique et stéaridonique estérifiés en position 1, 2 ou 3, de préférence 2, desdits glycérides et,

b) on récupère le résidu non hydrolysé de la réaction enzymatique en séparant les acides gras libres produits.

On peut citer comme lipase utile dans le procédé selon l'invention les lipases Geotrichum candidum , Candida cylindraces , Candida paralipolytica , Rhizopus delemar ainsi que la lipase pancréatique de porc.

EP 0 421 867 A1

FIG_1

(n-6)

C18:2 acide linoléique
↓ Δ6 désaturase
C18:3 acide gamma-linolénique
→ C20:3 acide dihomogrammalinolénique → PG E1
→ dérivés hydroxylés
Δ5 désaturase
prostacycline, thromboxane
PG E2
C20:4 acide arachidonique
→ leucotriènes dérivés hydroxylés
→ C22:4
↓ Δ4 désaturase
C22:5

(n-3)

C18:3 acide α-linolénique
↓ Δ6 désaturase
C18:4 acide stéaridonique
→ C20:4 acide éicosatétraénoique
Δ5 désaturase
PG E3
C20:5 acide éicosapentaénoïque (EPA)
→ C22:5 acide docosapentaénoïque (DPA)
↓ Δ4 désaturase
C22:6 acide docosahéxaénoïque (DHA)

2

La présente invention concerne un procédé de production d'un mélange de glycérides ou d'une matière grasse telle qu'une huile végétale en contenant, enrichis en acides gras gamma-linolénique et/ou stéaridonique sous forme estérifiée en position 1, 2 ou 3 de préférence 2 des glycérides.

La présente invention concerne également un procédé de production d'acides gamma-linolénique et/ou stéaridonique gras libres présentant une première insaturation en position delta 6.

Enfin, la présente invention concerne un mélange de glycérides enrichis en acide gras gamma-linolénique et/ou stéaridonique libres ou sous forme estérifiée en position 1, 2 ou 3 de préférence 2, des glycérides, ou une huile en contenant ou un mélange d'acide gras obtenu(e) par des procédés selon l'invention.

L'influence bénéfique d'une alimentation riche en acides linoléique et alphalinolénique, acides gras parents indispensables des familles d'acides gras jouant un rôle essentiel : - famille linoléique et alpha linolénique, est connue depuis plusieurs années. On a longtemps cru qu'il était seulement indispensable de fournir à l'homme ces deux acides gras parents, l'organisme humain étant présumé capable de synthétiser leurs dérivés supérieurs plus insaturés en C18, C20, C22, en toutes circonstances. L'expérience montre de plus en plus que ce n'est pas le cas. Il est des situations en prévention ou en thérapeutique où il s'avère préférable de fournir directement ces dérivés supérieurs préformés. Ces acides gras en C18, C20, C22, présentant de 3 à 6 doubles liaisons de la famille linoléique et alphalinolénique sont de plus en plus reconnus comme conditionnellement indispensables, ("contitionnaly non dispensable").

On a décrit dans FR 2 490 631 des compositions lipidiques utilisables en diététique, réanimation et thérapeutique à base d'huiles enrichies en acide gamma-linolénique libres ou sous forme estérifiée, c'est-à-dire présent sous forme de triglycérides.

Les composés les plus intéressants de la matière grasse, de l'huile naturelle, végétale ou animale deviennent les acides gras polyinsaturés dérivant des acides gras essentiels alpha-linolénique (famille n-3) et linoléique (famille n-6).

De manière non exhaustive, on peut citer pour la famille n-6 :

| | | |
|---|---|---|
| l'acide gamma-linolénique | C18:3 | 6,9,12 |
| l'acide dihomo-gammalinolénique | C20:3 | 8,11,14 |
| l'acide arachidonique | C20:4 | 5,8,11,14 |
| l'acide docosatétraénoïque | C22:44 | 7,10,13,16 |
| l'acide docosapentaénoïque | C22:5 | 4,7,10,13,16 |

et pour la famille n-3 :

| | | |
|---|---|---|
| l'acide stéaridonique | C18:4 | 6,9,12,15 |
| l'acide eicosapentaénoïque | C20:5 | 5,8,11,14,17 |
| L'acide docosahexaénoïque | C22:6 | 4,7,10,13,16,19 |

En effet, les acides gras essentiels sont nécessaires pour la formation des membranes et pour la régénération des icosanoïdes (J.L. BEARE-ROGERS, Revue Française des Corps Gras, 32, 1, 3-9).

En ce qui concerne les dérivés de l'acide gamma-linolénique, les acides dihomogammalinolénique et arachidonique sont des précurseurs des icosanoïdes cycliques (prostaglandines E1, E2, prostacycline, thromboxane) et linéaires (acides gras hydroxylés et leucotriènes).

La prostaglandine E1 (PG E1) et la prostacycline sont des dérivés anti-agrégants tandis que la PG E2 et le thromboxane possèdent des propriétés proagrégantes. Ces icosanoïdes interviennent également dans la régulation de la pression artérielle.

Les huiles végétales de type bourrache, onagre, et pépins de cassis, connaissent actuellement un développement spectaculaire. L'apport en acide gamma-linolénique qu'elles constituent permet de palier aux déficiences de l'appareil enzymatique de synthèse des icosanoïdes. (A. UZZAN, Rev. Française des Corps Gras, 33, 10, 385-389).

Une fois les problèmes de culture résolus, parce qu'elle constitue la source la plus riche en acide gamma-linolénique, l'huile de bourrache sera sans doute la plus répandue sur le marché. Elle titre de 18 à 25 % (de ses acides gras totaux) en acide gamma-linolénique.

La préparation d'une huile contenant de l'acide gamma-linolénique enrichie en acide gamma-linolénique a été proposée dans FR 2 490 631 en réalisant une hydrolyse chimique ou enzymatique des triglycérides contenus dans l'huile puis en séparant les acides gras obtenus et resynthétisant les triglycérides enrichis uniquement en acide gamma-linolénique.

Le brevet FR 2 515 174 propose la synthèse chimique de triglycérides particuliers où les acides gras polyinsaturés sont introduits en position 2 sur le glycérol.

La préparation d'une huile enrichie en acide gamma-linolénique est proposée en utilisant la complexation des acides gras polyinsaturés dans l'urée (ACKMAN et al., JAOCS, 65, 1, 136-138) et la cristallisation fractionnée proposée par SUZUKI (brevet WO 86/04354).

Une classe d'enzyme appelée lipase est connue entre autres pour hydrolyser les liaisons esters unissant les résidus acyles des acides gras au noyau glycéridique en position 1, 2 ou 3 et libérer les acides gras correspondants.

Parmi toutes les lipases, on connaît différentes classes qui se distinguent par des modalités de lipolyse différentes.

Ainsi P.E. SONNET (JAOCS, 65, 6, 900-904) distingue des :

- lipases non spécifiques qui hydrolysent les acides gras quels que soient leurs positions sur le glycérol et leurs nombres d'atomes de carbone.

- lipases qui hydrolysent sélectivement les acides gras selon leur position sur le glycérol. On connaît en particulier des lipases 1, 3 spécifiques telles que la lipase pancréatique de porc ou la lipase de Rhizopus arrhizus qui hydrolysent sélectivement les acides gras en position 1 et 3 sur le glycérol.

- lipases qui hydrolysent sélectivement les acides gras selon leur nature. Ainsi, les lipases produites par Candida rugosa ou par Aspergillus niger sont connues pour hydrolyser préférentiellement l'acide oléique, parce qu'il possède une double liaison en position delta 9.

Le but de la présente invention est de fournir un procédé simplifié d'enrichissement d'une matière grasse telle qu'une huile d'origine végétale, en ses acides gras polyinsaturés intéressants dérivés des acides linoléique et alphalinolénique tels que définis ci-dessus en leur conservant leur position naturelle sur leur triglycéride d'origine, et d'accroître la valeur thérapeutique préventive ou de bien-être de ladite matière grasse.

En particulier un but de la présente invention est de proposer un procédé d'enrichissement simplifié d'huile végétale notamment d'huile de bourrache enrichie en acides gamma-linolénique.

La découverte qui est à la base du procédé objet de la présente invention est la non sélectivité que possèdent certaines lipases à l'égard de certaines positions de l'insaturation des acides gras du substrat glycéridique. Pour ces lipases l'hydrolyse se produit sauf pour certains acides gras donnés. Plus précisément, on a découvert que les acides gras dont la première double liaison se situe à 6 atomes de carbone de l'extrémité carboxylique présents dans l'huile végétale (ou estérifié sous forme de glycéride) (position delta 6), résistent à la lipolyse pour une certaine classe de lipases.

La présente invention a donc pour objet un procédé de production par sélection d'un mélange de glycérides enrichis en acides gras gamma-linolénique et/ou stéaridonique sous forme estérifiée en position 1, 2 ou 3, de préférence 2, des glycérides ou d'une matière grasse telle qu'une huile en contenant, caractérisé en ce que :

a) à partir d'un mélange de glycérides dont certains contiennent naturellement de l'acide gras gamma-linolénique et/ou stéaridonique sous forme estérifiée en position 1, 2 ou 3, de préférence 2, sur le glycéride, ou d'une matière grasse telle qu'une huile végétale en contenant, on hydrolyse ledit mélange ou l'huile en contenant avec une lipase dont la spécificité est de ne pas ou difficilement hydrolyser le lien ester des acides gras gamma-linolénique et stéaridonique estérifiés en position 1, 2 ou 3 desdits glycérides et,

b) on récupère le résidu non hydrolysé de la réaction enzymatique en séparant les acides gras libres produits.

Par hydrolyse "difficile", on entend notamment que la cinétique d'hydrolyse est plus lente que pour d'autres positions de double liaison.

De préférence, on utilisera un mélange de glycérides présentant des acides gras à insaturation en position delta sous forme estérifiée en position 2 qui est leur position naturelle la plus fréquente.

Parmi les enzymes utiles dans le procédé selon l'invention, on peut citer de préférence des lipases produites par des microorganismes eucariotes, telles que des lipases de levures ou de moisissures ou des lipases produites par des organismes eucariotes supérieurs telle que la lipase pancréatique de porc.

Il est préférable d'utiliser des lipases non spécifiques vis-à-vis de la position de l'acide gras sur le glycérol afin que l'hydrolyse soit la plus complète possible.

Parmi les lipases de levures, on peut citer plus particulièrement Geotrichum candidum , Candida cylindracea , Candida paralipolytica , comme lipases de moisissures, on peut citer Rhizopus delemar .

On peut sélectionner les organismes lipolytiques appropriés selon l'invention en utilisant un milieu gélosé contenant de la tributyrine.

Les organismes producteurs de lipase se caractérisent par un halo clair qui entoure les colonies (Roblain D., Destain J. and Thonart Ph., Belgian Journal of Food Chemistry and Biotechnology, 44, 3, 1989). Les différentes lipases sont ensuite produites en fermenteur par la culture de chacune des souches sélectionnées. Afin de tester leur éventuelle "non-spécificité" à l'égard de l'insaturation du substrat, on peut réaliser des réacteurs enzymatiques comme décrit dans l'exemple 1. Néanmoins, on peut aussi mettre au point un test spécifique pour ce type de lipase, qui remplace la production d'enzyme et la réalisation des réacteurs. Ce test consiste à produire un substrat spécifique composé d'un triglycéride ayant comme seul acide gras, celui qu'on ne veut pas hydrolyser ou plus simplement, un ester de cet acide avec un alcool bien choisi. Dans ce cas, et pour autant que ce substrat soit non toxique pour la souche, les colonies lipolytiques intéressantes, c'est-à-dire productrices d'une lipase ayant les caractéristiques recherchées, sont incapables d'hydrolyser ce substrat et donc de le métaboliser. Il en résulte une non ou très faible croissance de ces souches sur milieu gél osé contenant le substrat spécifique comme seule source de carbone.

Toutes les huiles végétales contenant naturellement un ou plusieurs acides gras gamma-linolénique et/ou stéaridonique sous forme libre ou sous forme de glycérides conviennent pour le procédé selon l'invention. Actuellement, sur le marché, il existe seulement quelques huiles répondant à ce critère et facilement disponibles. Il s'agit plus particulièrement de l'huile végétale notamment de bourrache ou de pépins de cassis pour l'acide stéaridonique, et l'huile de bourrache, l'huile d'onagre ou primrose, huile de pépin de cassis pour l'acide gamma-linoléique.

Il faut également mentionner les huiles produites par des organismes manipulés génétiquement.

Le procédé d'enrichissement selon l'invention permet d'atteindre une teneur en acide gamma-linolénique et/ou stéaridonique dans sa position naturelle sur le glycéride, par exemple, de plus du double de sa valeur initiale. Ainsi, de façon très avantageuse, la quantité journalière d'acide gras prescrit par le nutritionniste peut être augmentée sans modifier le volume de la prise, ou la biodisponibilité de l'acide gras.

Dans un mode de réalisation particulier du procédé selon l'invention, la lipase est mise en solution dans un tampon et agitée en présence du mélange de glycérides ou de l'huile à enrichir.

Le choix de la solution tampon se fonde sur l'analyse des courbes décrivant l'activité de l'enzyme en fonction du pH et de la stabilité de l'enzyme à un pH donné au cours du temps. La lipase est mise en solution dans un tampon à un pH donné où son activité et sa stabilité sont optima. En général, le pH est compris entre 3 et 10.

Avantageusement, la réaction d'hydrolyse doit être stoppée et le résidu non hydrolysé récupéré lorsque le temps optimum est atteint au-delà duquel l'hydrolyse se produit aussi sur les insaturations en position autre.

A titre d'exemples non limitatifs de conception du réacteur utile pour le procédé selon l'invention, celui-ci comportera 50 % de l'huile choisie comme substrat et 50 % de la solution enzymatique, au lieu de la réaction.

L'ensemble sera de préférence agité et thermostatisé à la température optimale de l'enzyme réalisant le compromis le plus avantageux entre l'activité et la stabilité enzymatique optimales, en général entre 15 et 65° C.

La matière grasse obtenue après en général 2 à 6 heures de réaction est fractionnée en mélange de glycérides d'une part, et en acide gras libre d'autre part.

La séparation des acides gras libres des glycérides peut se faire par solubilisation des acides gras libres en phase aqueuse par saponification, par exemple par KOH.

L'ajout de KOH est alors réalisée dans des conditions douces afin de s'assurer que seuls les acides gras libres, c'est-à-dire non estérifiés au glycérol sont saponifiés et solubilisés dans la phase aqueuse. Les glycérides non touchés par cette réaction demeurent dans la phase organique. Les fractions ainsi obtenues sont analysées en chromatographie en phase gazeuse après transestérification au MeOH/BF$_3$. Les compositions en acides gras sont ainsi révélées.

La quantité d'enzymes à introduire dans le réacteur par gramme d'huile à hydrolyser sera fonction de la teneur initiale de l'huile en acides gras d'intérêt et de l'activité lipolytique de l'enzyme. Par exemple, cette concentration sera comprise entre 0,1 et 100 mgr/gr d'huile. Mai bien entendu, une quantité inférieure pourra être utilisée auquel cas la réaction devra durer plus longtemps et de même une quantité supérieure ' pourra être utilisée mais le facteur économique constitue un facteur limitant dans ce cas ci.

· Enfin, signalons que l'homme de l'art pourra avoir recours aux biohydrolyses en réacteur à enzymes immobilisées, ou à membrane, pour permettre la séparation de la fraction partiellement hydrolysée, des acides gras libérés.

La présente invention a également pour objet un procédé de production d'acides gras gamma-linolénique et/ou stéaridonique libres caractérisé en ce qu'on effectue l'hydrolyse par toutes méthodes connues de l'homme de l'art notamment chimique du mélange glycéridique enrichi ou de la matière grasse enrichie par un procédé selon l'invention, et on récupère les acides gras libres.

La présente invention a enfin pour objet un mélange de glycérides enrichis en acides gras gamma-linolénique et/ou stéaridonique sous forme estérifiée en position 1, 2 ou 3 de préférence 2 des glycérides ou une huile en contenant obtenu(e) par le procédé selon l'invention, ou encore un mélange de glycérides enrichis en acide gras gamma-linolénique et/ou stéaridonique sous forme libre issu de l'hydrolyse complète du mélange glycéridique enrichi selon l'invention.

Les acides gras gamma-linolénique et/ou stéaridonique libres issus de l'hydrolyse complète du mélange glycéridique enrichi, objet de l'invention peuvent également permettre de reconstituer des triglycérides nouveaux caractérisés par un taux élevé d'acides gras polyinsaturés en particulier en position 2 sur le glycérol. De ce fait, ils assureront une meilleure absorption par l'organisme des acides gras libres polyinsaturés et augmenteront la valeur thérapeutique des huiles proposées.

D'autres caractéristiques et avantages de la présente invention apparaîtront à la lumière des exemples qui vont suivre, lesquels illustrent l'invention sans toutefois la limiter.

**EXEMPLE 1 : Hydrolyse d'une huile de bourrache par la lipase de Candida cylindracea**

a) Détermination de l'activité de la lipase

Celle-ci est réalisée par titration directe des acides gras libérés dans le milieu. La détermination se fait en présence d'un substrat spécifique à l'enzyme considérée et dans des conditions rigoureusement définies.

Pour rappel, une unité d'activité lipolytique (U) est la quantité d'enzyme qui libère un microéquivalent d'acide gras par minute dans les conditions de l'expérience.

En l'occurence, le substrat est une émulsion réalisée à partir d'une huile d'olive neutralisée et stabilisée par ajout d'une solution à 2 % de polyvinylalcool.

L'enzyme, mise en solution dans un tampon (correspondant à son pH optimal), est incubée durant 15 minutes à 37° C avec le substrat.

Après quoi, la réaction est stoppée et les acides gras libérés sont titrés par une solution de NaOH 0,05 N en présence de phénolphtaléine.

La lipase produite par Candida cylindracea titre à 10.000 U/gr en solution dans un tampon citrate pH 5,6.

b) Réacteurs proprement dits

Le réacteur se compose à :
50 % de la solution enzymatique
50 % de l'huile-substrat.

La solution enzymatique est réalisée par dissolution de 100 mgr d'enzyme dans 20 g de tampon citrate (pH 5,6).

Ceci correspond à une concentration enzymatique de 5 mgr enzyme/gr d'huile, soit 50 U/gr d'huile.

Ensuite, 20 g d'huile de bourrache (à 17,7 % d'acide gamma linolénique) sont ajoutés. L'ensemble est agité et thermostatisé à 37° C.

Des échantillons de 1 ml de milieu sont prélevés au bout de 0,5 ; 1 ; 2 ; 3 ; 4 ; 5 et 6 heures afin de mesurer la valeur du DH. Après 2, 4 et 6 heures, ces échantillons sont doublés afin de pouvoir déterminer l'évolution de la teneur en acide gamma-linolénique.

c) Evaluation du degré d'hydrolyse

Le calcul du degré d'hydrolyse (DH) se fait selon la formule suivante :

$$DH = \frac{\text{quantité d'acides gras libérés (méq)}}{\text{quantité théorique d'acides gras (méq)}} \times 100$$

La quantité d'acides gras libérés est calculée par titrage de la matière grasse à l'aide de soude 0,05 N. La quantité théorique de matière d'acide gras est définie ainsi :

$$\frac{\text{poids de MG dans l'échantillon}}{\text{poids moléculaire moyen des acides gras}} \times 1000$$

Le poids moléculaire moyen est calculé au départ de la composition en acides gras du substrat de l'enzyme.

Afin de déterminer la quantité d'acides gras libérés, on prélève 1 ml d'échantillon aux temps t = 0, t = 30 min, t = 1 heure et ainsi de suite, chaque heure durant les six premières heures. Un dernier échantillon est prélevé après une nuit afin de définir un degré d'hydrolyse proche du maximum.

Chaque échantillon contient 50 % d'huile, ce qui correspond approximativement à 0,5 gr.

Ils sont alors additionnés de 9 ml d'un mélange 50:50 d'alcool/acétone en vue de dénaturer la lipase.

Ensuite on titre les acides gras libérés par une solution 0,05 N de NaOH en présence de phénolphtaléine. Dans ces conditions, la quantité d'acides gras libérés vaut :

[0,05 x (vol en ml de NaOH 0,05 N nécessaire pour titrer les acides gras libres)] milliéquivalents.

La quantité théorique présente dans 0,5 gr de matière grasse tient compte du poids moléculaire moyen des acides gras de l'huile-substrat.

Pour chacune des huiles, on calcule cette valeur au départ de la composition centésimale relative en acide gras.

d) Détermination de la teneur en acide gamma-linolénique du mélange de glycérides

A partir du mélange de lipides neutres que constitue la matière grasse prélevée en cours de lipolyse, on sépare les acides gras libres par formation de sels hydrophiles.

Pour cela, on ajoute 10 ml de EtOH à 50 % et 0,5 ml de KOH à 50 % à environ 1 gr de matière grasse. Le mélange est agité durant une heure à 4° C.

Après décantation, les acides gras libres devenus hydrophiles par saponification se trouvent dans la phase aqueuse.

Par contre, le mélange de tri-, di- et mono-glycérides non touchés par cette réaction demeure hydrophobe.

Il est alors récupéré et soumis à une transestérification au MeOH/BF$_3$.

Par cette réaction, les glycérides sont hydrolysés et les résidus acyls des acides gras sont transformés en leurs esters méthyliques volatils.

De manière détaillée, le mélange à base de trifluorure de bore est ajouté à environ 100 mg de matière grasse ainsi que 0,2 ml d'hexane. Les tubes, hermétiquement fermés, sont placés au bain-marie à 70° C durant une heure et demi.

Ensuite, 0,5 ml de NaCl saturé et 0,2 ml d'H$_2$SO$_4$ à 10 % sont ajoutés.

Après homogénéisation, l'échantillon est éventuellement dilué dans 5 à 10 ml d'hexane.

L'analyse par chromatographie en phase gazeuse est alors réalisée sur colonne capillaire de type FFAP (Free Fatty Acid Phase).

Les résultats obtenus permettent de dresser le tableau suivant :

7

EP 0 421 867 A1

Tableau 1

| Temps (heures) | % DH | Teneur en acide gamma-linolénique du mélange de glycérides |
|---|---|---|
| 0 | 0 | 17,7 |
| 0,5 | 21,8 | 32,0 |
| 1 | 35,9 | 31,7 |
| 2 | 42,2 | 39,0 |
| 3 | 46,1 | 39,2 |
| 4 | 46,6 | 46,9 |
| 16 | 51,3 | 42,8 |

Les résultats du tableau 1 montrent que on atteint un taux en acide gamma-linolénique de 47 % dans la fraction glycéridique.

Afin de compléter cet exemple, on présente au tableau 2 la teneur en acide gamma-linolénique de la fraction acides gras libres.

Tableau 2

| Temps (heures) | % DH | Teneur en gamma-linolénique des acides gras libres |
|---|---|---|
| 0 | 0 | 4,7 |
| 0,5 | 21,8 | 5,7 |
| 1 | 35,9 | 3,7 |
| 2 | 42,2 | 3,3 |
| 3 | 46,1 | 4,7 |
| 4 | 46,6 | 3,8 |
| 16 | 51,3 | 7,1 |

Les faibles pourcentages en acide gamma-linolénique obtenus pour la fraction issue de la lipolyse confirment la difficulté avec laquelle la lipase de Candida cylindracea hydrolyse les acides gras à insaturation en position 6.

Par ailleurs, la teneur en acide gamma-linolénique dans la fraction glycéridique semble atteindre un maximum après 4 heures de réactions. Par la suite, l'hydrolyse se poursuit puisque le DH augmente encore mais le pourcentage relatif d'acide gamma-linolénique diminue. Vraisemblablement la spécificité à l'égard de la position de l'insaturation n'est pas totale et au delà de 4 heures, la lipase hydrolyse également l'acide gamma-linolénique.

## EXEMPLE 2

Cet exemple est identique à l'exemple 1 si ce n'est que l'huile utilisée comme substrat de l'enzyme est une huile d'"Evening Primrose Oil" (EPO) qui titre à 9,9 % d'acide gamma-linolénique.

Le réacteur est donc constitué de :

50 % d'une solution enzymatique contenant 100 mgr de lipase produite par Candida cylindracea dans 20 g de tampon citrate à pH 5,6, soit une concentration de 50 U/gr d'huile

50 % d'huile d'EPO à 9,9 % d'acide gamma-linolénique.

On obtient les résultats suivants :

8

Tableau 3

| Temps (heures) | % DH | Teneur en acide gamma-linolénique du mélange de glycérides |
|---|---|---|
| 0 | 0 | 9,9 |
| 0,5 | 21,8 | 13,7 |
| 1 | 35,9 | 18,3 |
| 2 | 42,2 | 29,8 |
| 3 | 46,1 | 31,9 |
| 4 | 46,6 | 36,4 |

## EXEMPLE 3

On utilise à nouveau l'huile de bourrache. Toutefois, celle-ci titre à 20,2 % d'acide gamma-linolénique (PM moyen = 269 g). La lipase est toujours celle produite par Candida cylindracea (10.000 U/g).

Le réacteur a donc la composition suivante :

50 % de solution enzymatique de Candida cylindracea contenant 200 mg d'enzyme dans 20 g de tampon pH 5,6, soit une concentration de 100 U/gr d'huile.

50 % d'huile de bourrache à 20,2 % d'acide gamma linolénique.

Ces conditions donnent lieu aux résultats suivants :

Tableau 4

| Temps (heures) | % DH | Teneur en gamma du mélange de glycérides |
|---|---|---|
| 0 | 0 | 20,2 |
| 0,5 | 24 | - |
| 1 | 35 | - |
| 2 | 40 | 38,7 |
| 3 | 49 | - |
| 4 | 53 | 45,8 |
| 6 | 57 | 43,3 |

## EXEMPLE 4

On utilise une lipase produite par Candida paralipolytica. Celle-ci a une activité de 15.000 U/g à pH 7, soit une concentration de 100 U/gr d'huile.

L'huile-substrat est une huile de bourrache à 20,2 % d'acide gamma-linolénique. Ces nouvelles modifications constituent le réacteur suivant :

50 % de solution enzymatique réalisée avec 200 mgr d'enzyme dans 20 g de tampon phosphate à pH 7.

50 % d'huile de bourrache à 20,0 % d'acide gamma linolénique.

Les résultats sont repris dans le tableau suivant :

EP 0 421 867 A1

Tableau 5

| Temps (heures) | % DH | Teneur en acide gamma linolénique du mélange de glycérides |
|---|---|---|
| 0 | 0 | 20,2 |
| 0,5 | 3 | - |
| 1 | 7,5 | - |
| 2 | 17 | 24,8 |
| 4 | 27 | 26,6 |
| 6 | 32 | 28,4 |

Les teneurs en gamma plus faibles après 4 heures de réaction qu'elles ne l'étaient dans l'exemple précédent s'expliquent par le fait que le DH atteint dans ce laps de temps est bien moins élevé avec cette nouvelle lipase. Toutefois, en aucun cas, la non-spécificité d'hydrolyse ne peut être mise en cause.

## EXEMPLE 5

On utilise la lipase produite par Geotrichum candidum . Son activité lipasique atteint 8.000 U/gr à pH 7.
La composition du réacteur est la suivante :
50 % de solution enzymatique à 500 mgr d'enzyme dans 20 g de tampon pH 7, soit une concentration de 200 U/gr d'huile.
50 % d'huile de bourrache à 20,2 % d'acide gamma (PM moyen des acides gras = 269 gr).
Les résultats sont repris dans le tableau suivant :

Tableau 6

| Temps (heures) | % DH | Teneur en acide gamma-linolénique du mélange de glycérides |
|---|---|---|
| 0 | 0 | 20,2 |
| 0,5 | 15,3 | - |
| 1 | 21,8 | - |
| 2 | 30,9 | 31,1 |
| 3 | 36,6 | - |
| 4 | 39,3 | 33,5 |
| 5 | - | - |
| 6 | 42,8 | 33,8 |

Ici encore, les valeurs plus faibles de pourcentage en acide gamma-linolénique sont dues à une hydrolyse moins poussée de l'huile-substrat.

## EXEMPLE 6

Après avoir poursuivi tous nos tests en fiole de 100 ml, on a réalisé les réacteurs dans des cuves agitées de 1 litre.
La composition est la suivante :
50 % de solution enzymatique de Candida cylindracea contenant 5 gr d'enzyme dans 500 gr de tampon citrate pH 5,6, soit une concentration de 100 U/gr d'huile.
50 % d'huile de bourrache.
Les résultats sont repris au tableau 7.

EP 0 421 867 A1

Tableau 7

| Temps (heures) | % DH | Teneur en acide gamma-linolénique du mélange de glycérides |
|---|---|---|
| 0 | 0 | 19,9 |
| 0,5 | 25,4 | 38,2 |
| 1 | 30,8 | 45,6 |
| 2 | 35,4 | 48,7 |
| 3 | 39,1 | - |
| 4 | 39,4 | 46,4 |
| 5 | 41,0 | - |
| 24 | 50,3 | - |

Ces résultats sont intéressants car ils montrent l'influence du scale-up sur l'efficacité de la réaction enzymatique.

En effet, pour une concentration enzymatique identique à celle de l'exemple 3, on atteint 49 % d'acide gamma-linolénique en 2 heures et pour 38 % d'hydrolyse.

Par ailleurs, l'exemple montre qu'au delà de ce temps optimal de réaction, la teneur en acide gamma-linolénique régresse au sein du mélange de glycérides enrichis.

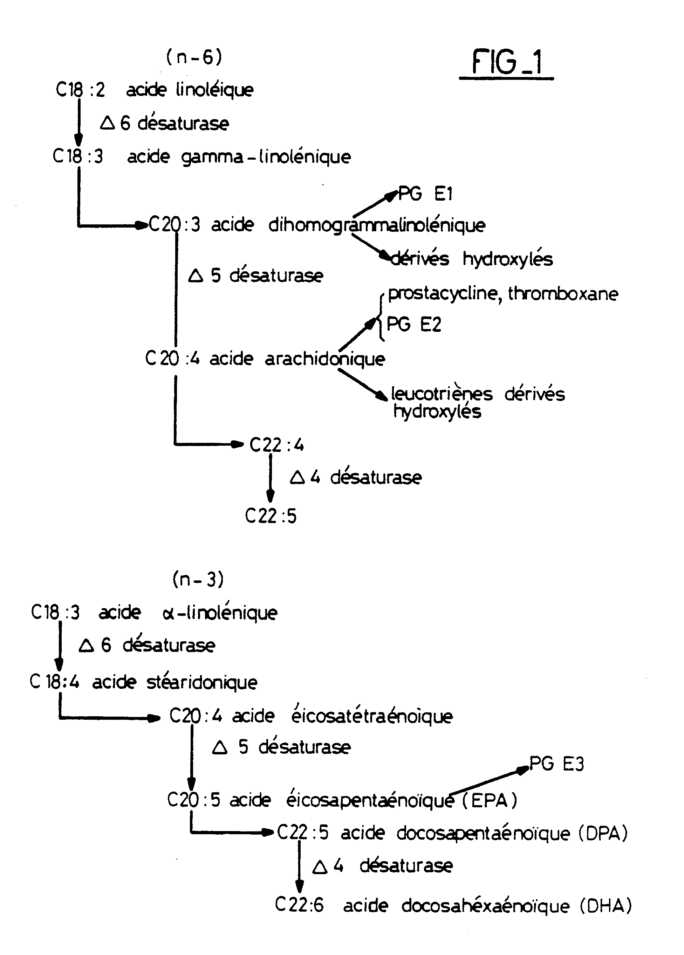

**Revendications**

1. Procédé de production par sélection d'un mélange de glycérides enrichis en acide gras gamma-linolénique et/ou stéaridonique sous forme estérifiée en position 1, 2 ou 3 de préférence 2, des glycérides, ou d'une matière grasse telle qu'une huile végétale en contenant, caractérisé en ce que :
a) à partir d'un mélange de glycérides dont certains contiennent naturellement des acides gras gamma-linolénique et/ou stéaridonique sous forme estérifiée en position 1, 2 ou 3 de préférence en position 2 sur le glycéride, ou de la matière grasse en contenant, on hydrolyse ledit mélange ou la matière grasse en contenant avec une lipase dont la spécificité est de ne pas ou difficilement hydrolyser le lien ester des acides gras gamma-linolénique et stéaridonique estérifiés en position 1, 2 ou 3 desdits glycérides,
b) on récupère le résidu non hydrolysé de la réaction enzymatique en séparant les acides gras libres produits.

2. Procédé de production par sélection d'un mélange de glycérides enrichis en acide gras gamma-linolénique et/ou stéaridonique ou d'une matière grasse en contenant selon la revendication 1, caractérisé en ce que la lipase est une lipase produite par des microorganismes eucariotes tels que des lipases de levures et de moisissures ou des lipases produites par des organismes eucariotes supérieurs tels que la lipase pancréatique de porc.

3. Procédé selon la revendication 2, caractérisé en ce que la lipase est produite par Geotrichum candidum , Candida cylindracea , Candida paralipolytica , Rhizopus delemar .

4. Procédé selon la revendication précédente, caractérisé en ce que l'huile est de l'huile de bourrache, de l'huile d'onagre ou primrose, de l'huile de pépin de cassis.

5. Procédé selon l'une des revendications précédentes, caractérisé en ce que le réacteur se compose de 50 % en poids de la solution enzymatique, et 50 % d'huile, au lieu de la réaction.

6. Procédé de production d'acides gras gamma-linolénique et/ou stéaridonique libres, caractérisé en ce qu'on effectue l'hydrolyse notamment chimique du mélange glycéridique enrichi ou de l'huile enrichie selon l'une des revendications précédentes et on récupère les acides gras libres.

7. Glycérides enrichis en acides gras gamma-linélonique et/ou stéaridonique libres ou sous forme estérifiée en position 1, 2 ou 3, de préférence 2, des glycérides ou acides gras gamma-linolénique et/ou stéaridonique libres ou matière grasse en contenant obtenu(e) par un procédé selon l'une des revendication précédentes.

11

FIG_1

(n-6)

C18:2 acide linoléique

Δ 6 désaturase

C18:3 acide gamma-linolénique

C20:3 acide dihomogrammalinolénique → PG E1
→ dérivés hydroxylés

Δ 5 désaturase

prostacycline, thromboxane
PG E2

C20:4 acide arachidonique

leucotriènes dérivés hydroxylés

C22:4

Δ 4 désaturase

C22:5

(n-3)

C18:3 acide α-linolénique

Δ 6 désaturase

C18:4 acide stéaridonique

C20:4 acide éicosatétraénoique

Δ 5 désaturase

PG E3

C20:5 acide éicosapentaénoïque (EPA)

C22:5 acide docosapentaénoïque (DPA)

Δ 4 désaturase

C22:6 acide docosahéxaénoïque (DHA)

Office européen
des brevets

RAPPORT DE RECHERCHE
EUROPEENNE

Numéro de la demande

**EP 90 40 2720**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. CI.5) |
|---|---|---|---|
| X | PATENT ABSTRACTS OF JAPAN, vol. 7, no. 289 (C-202)[1434], 23 décembre 1983; & JP-A-58 165 796 (ASAHI DENKA KOGYO K.K.) 30-09-1983 * Résumé * | 1-4,7 | C 11 C 1/04 C 12 P 7/64 |
| X | CHEMICAL ABSTRACTS, vol. 104, no. 23, juin 1986, page 605, résumé no. 205559b, Columbus, Ohio, US; & JP-A-60 234 590 (ASAHI DENKA KOGYO K.K.) 21-11-1985 * Résumé * | 1-5,7 | |
| X | CHEMICAL ABSTRACTS, vol. 105, no. 5, août 1986, page 610, résumé no. 41278b, Columbus, Ohio, US; & JP-A-61 15 692 (KAO CORP.) 23-01-1986 * Résumé * | 1,2,7 | |
| X | CHEMICAL ABSTRACTS, vol. 105, no. 5, août 1986, page 610, résumé no. 41279c, Columbus, Ohio, US; & JP-A-61 15 693 (KAO CORP.) 23-01-1986 * Résumé * | 1,2,7 | |
| A | FETTE SEIFEN ANSTRICHMITTEL, vol. 88, no. 9, septembre 1986, pages 365-367, Leinfelden-Echterdingen, DE; O. LIE et al.: "Fatty acid specificity of candida cylindracea lipase" * Page 366 * | 1-4 | **DOMAINES TECHNIQUES RECHERCHES (Int. CI.5)** C 11 C C 12 P |

Le présent rapport de recherche a été établi pour toutes les revendications

| Lieu de la recherche | Date d'achèvement de la recherche | Examinateur |
|---|---|---|
| La Haye | 04 janvier 91 | LEPRETRE F.G.M.J. |

CATEGORIE DES DOCUMENTS CITES
X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention

E : document de brevet antérieur, mais publié à la date de dépôt ou après cette date
D : cité dans la demande
L : cité pour d'autres raisons
 
& : membre de la même famille, document correspondant